(19) Europäisches Patentamt
European Patent Office
Office européen des brevets

(11) Veröffentlichungsnummer: **0 496 370 A2**

(12) # EUROPÄISCHE PATENTANMELDUNG

(21) Anmeldenummer: **92100997.3**

(51) Int. Cl.$^5$: **C07C 205/37**, C07C 201/12

(22) Anmeldetag: **22.01.92**

(30) Priorität: **23.01.91 DE 4101808**

(43) Veröffentlichungstag der Anmeldung: **29.07.92 Patentblatt 92/31**

(84) Benannte Vertragsstaaten: **CH DE FR GB IT LI**

(71) Anmelder: **HOECHST AKTIENGESELLSCHAFT**
**Postfach 80 03 20**
**W-6230 Frankfurt am Main 80(DE)**

(72) Erfinder: **Folz, Georg, Dr.**
**Rauenthaler Weg 32**
**W-6000 Frankfurt am Main 71(DE)**
Erfinder: **Papenfuhs, Theodor, Dr.**
**Heinrich-Bleicher-Strasse 40**
**W-6000 Frankfurt am Main 50(DE)**
Erfinder: **Schubert, Hans, Dr.**
**Hölderlinstrasse 56**
**W-6233 Kelkheim (Taunus)(DE)**

(54) **Verfahren zur Herstellung von o-Nitrophenetol.**

(57) Die Erfindung betrifft ein Verfahren zur Herstellung von o-Nitrophenetol durch Umsetzung von o-Nitrochlorbenzol mit geringem Überschuß an Ethanol in 45 bis 55 gew.-%iger Alkalimetallhydroxydlösung in Gegenwart eines Phasentransfer-Katalysators bei Temperaturen von 55 bis 70 °C, dadurch gekennzeichnet, daß die Ethanolkonzentration in der organischen Phase während des gesamten Reaktionsverlaufes 6 Gew.-%, bezogen auf die organische Phase, nicht übersteigt, daß man nach der Reaktion von gebildetem Salz abfiltriert, das Filtrat in o-Nitrophenetol und Mutterlauge trennt, und diese nach Aufkonzentrierung und Ausgleich des Verbrauchs wieder einsetzt.

Der Anfall von belastetem Abwasser ist erfindungsgemäß sehr gering.

EP 0 496 370 A2

Die Erfindung betrifft ein verbessertes Verfahren zur Herstellung Von o-Nitrophenetol, das praktisch frei ist von Dichlorazoxybenzol und o-Nitrophenol, durch Ethoxylierung von o-Nitrochlorbenzol unter phasentransferkatalytischen Bedingungen und Verbrauch der Komponenten in praktisch stöchiometrischen Mengen und demgemäß ohne Anfall von belastetem Abwasser und umweltrelevanten Stoffen.

Das o-Nitrophenetol ist ein Vorprodukt zur Herstellung von o-Phenetidin, das wiederum ein wichtiges Zwischenprodukt zur Herstellung von Farbstoffen und Pharmazeutika darstellt.

In der Literatur sind bereits eine Reihe von Verfahren zur Herstellung von o-Nitrophenetol durch Umsetzung von o-Nitrochlorbenzol mit Ethanol in Gegenwart von Alkalien beschrieben worden (US-PS 2.545.597, GB-PS 902.306, C.A. 34, 5423 (1940), US-PS 3.085.113, J. scient. ind. Res. India 4 (1945) 369 bzw. 5 B (1946) 25). Hauptsächlicher Nachteil dieser Verfahren ist dabei das verstärkte Auftreten von Nebenprodukten, wie Azoxyverbindungen und o-Chloranilin, aus reduktiven Prozessen sowie von o-Nitrophenol. Um die Bildung von Azoxyverbindungen zu unterdrücken, wurde versucht, die Umsetzung in Gegenwart von Luft oder anderen Oxidationsmitteln, z.B. Schwermetalloxiden, durchzuführen (US-PS 2.545.597). Dennoch konnten diese Nebenreaktionen nie ganz unterdrückt und auch kein annähernd quantitativer Umsatz erreicht werden.

Zur Vermeidung dieser Nachteile ist auch schon versucht worden, die Ethoxylierung von Nitrochlorbenzolen in Gegenwart von Phasentransfer-Katalysatoren,beispielsweise organischen quartären Ammoniumsalzen, durchzuführen (DE-OS 2.634.419, DE-OS 3.120.912), wobei aber jeweils bei einem Katalysatoreinsatz von 8,5 - 12,5 Gew.-% auch bis zu 10 % Dichlorazoxybenzol gebildet wurde. Die in diesen Literaturstellen beschriebenen Verfahren beziehen sich dabei ausschließlich auf die Herstellung von p-Nitrophenetol.

Lediglich in der DE-OS 3.307.164, ist neben der Herstellung des p-Isomeren, auch erstmals die Ethoxylierung von o-Nitrochlorbenzol in konzentrierter Natronlauge in Gegenwart eines Tetramethylammoniumsalzes beschrieben. Danach soll o-Nitrophenetol in Ausbeuten über 97 %, frei von Nebenprodukten, erhalten werden. Da aber die Ethoxylierung von o-Nitrochlorbenzol erfahrungsgemäß sehr viel stärker zur Bildung von Nebenprodukten führt, ist es sehr erstaunlich, daß das Verfahren zur Herstellung von p-Nitrophenetol einfach auf das o-Isomere übertragen werden könnte. Bei der Nacharbeitung dieser DE-OS 3.307 164 (Beispiel 3) konnte dann auch kein von Nebenprodukten freies o-Nitrophenetol erhalten werden; vielmehr wurden immer erhebliche Mengen an Dichlorazoxybenzol, o-Chloranilin, o-Nitrophenol und andere, nicht näher identifizierte Produkte gefunden. Wegen der Empfindlichkeit der Ethoxylierung von o-Nitrochlorbenzol speziell gegenüber reduktiven Prozessen und des damit verbundenen Entstehens von Nebenprodukten wird in der Technik o-Nitrophenetol daher im allgemeinen über o-Nitrophenol und anschließender Ethylierung mit Chlorethyl hergestellt. Nachteilig bei diesem Verfahren ist jedoch seine Zweistufigkeit, ein hoher Bedarf an Alkali und der Anfall beträchtlicher Mengen von Diethylether sowie von stark belastetem Abwasser.

Ein großer wirtschaftlicher und umweltrelevanter Nachteil aller bisher beschriebenen Verfahren ist der hohe Bedarf an nicht wiedereinsetzbaren Alkalien. Letztendlich werden dadurch die Verfahren deutlich verteuert und auch die Umwelt erheblich belastet, da die Alkaliüberschüsse entsprechend entsorgt werden müssen (Abwasser). Es ist zwar auch schon ein Verfahren zur Herstellung von o-Nitrophenetol auf Basis von o-Nitrochlorbenzol bekannt, bei dem ohne einen derartig hohen Alkaliüberschuß gearbeitet wird. Das dabei entstehende Alkalichlorid ist jedoch unter technischen Bedingungen praktisch nicht filtrierbar und muß durch Zugabe von Wasser von dem erhaltenen o-Nitrophenetol abgetrennt werden. Dadurch fallen erhebliche Mengen an entsprechend belastetem Abwasser an, und ein Wiedereinsatz des Alkalis ist damit nicht mehr möglich.

Aufgabe der vorliegenden Erfindung war es daher, ein Verfahren zur Herstellung von o-Nitrophenetol durch Ethoxylierung von o-Nitrochlorbenzol bereitzustellen, das die vorstehend beschriebenen Nachteile nicht besitzt und das insbesondere ein o-Nitrophenetol in hohen Ausbeuten ohne störende Verunreinigungen liefert, das ohne die belastenden hohen Alkalilaugen-Überschüsse sowie weitgehend ohne Anfall von belastetem Abwasser auskommt und ein Wiedereinsatz des Alkalis ermöglicht.

Die Lösung dieser Aufgabe gelingt erfindungsgemäß überraschenderweise durch ein Verfahren zur Herstellung von o-Nitrophenetol durch Umsetzung von o-Nitrochlorbenzol mit geringem Überschuß an Ethanol in 45 bis 55 gew.-%iger Alkalimetallhydroxydlösung in Gegenwart eines Phasentransfer-Katalysators bei Temperaturen von etwa 55 bis 70 °C, das dadurch gekennzeichnet ist, daß die Ethanolkonzentration in der organischen Phase während des gesamten Reaktionsverlaufes etwa 6 Gew.-%, bezogen auf die organische Phase, nicht übersteigt, daß man nach der Reaktion von gebildetem Salz abfiltriert, das Filtrat in o-Nitrophenetol und Mutterlauge trennt, und diese nach Aufkonzentrierung und Ausgleich des Verbrauchs wieder einsetzt.

Das insgesamt zur Anwendung gelangende Ethanol wird stets in geringem Überschuß eingesetzt und zwar zweckmäßigerweise im Verhältnis von etwa 1,05 bis ca. 1,4 Mol Ethanol pro Mol o-Nitrochlorbenzol; als vorteilhaft hat sich dabei ein Molverhältnis von ca. 1,1 bis 1,2 Mol Ethanol pro Mol o-Nitrochlorbenzol erwiesen.

Die Zugabe des Ethanols, getrennt oder gemeinsam mit dem Phasentransfer-Katalysator, kann so vorgenommen werden, daß man nach einem gewissen, durch einen Pilotansatz zu ermittelnden Zeitschema, oder durch laufende Kontrolle (z.B. durch Gaschromatographie) der Ethanolkonzentration in der organischen Phase, diese nicht über etwa 6 Gew.-% steigen läßt und vorzugsweise unterhalb von 5 Gew.-%, jeweils bezogen auf die organische Phase, hält. Besonders bevorzugt liegt diese Ethanolkonzentration zwischen 1 und 3 Gew.-%. Die Reaktion läuft zu Beginn schneller, gegen Ende langsamer ab; entsprechend wird die Zudosierung reguliert. Im allgemeinen werden 8 bis 10 Stunden Zudosierzeit benötigt, wobei eine effektive Rührung erforderlich ist. Danach wird in der Regel noch einige Zeit, vorzugsweise bis zu 10 Stunden nachgerührt.

Als Alkalimetallhydroxide kommen für das erfindungsgemäße Verfahren vor allem Natrium- oder Kaliumhydroxid, insbesondere Natriumhydroxid, in Frage; auch Gemische davon sind geeignet. Bevorzugt wird das Alkalimetallhydroxid in Form einer 47 bis 52 %igen, speziell 50 %igen Lösung eingesetzt. Das Alkalimetallhydroxid wird zweckmäßigerweise in einer Menge von 3 bis 6 Mol, vorzugsweise 3,5 bis 5,5 Mol und insbesondere 4,375 bis 4,5 Mol, jeweils pro Mol o-Nitrochlorbenzol verwendet. Die nach der Reaktion durch Abfiltrieren vom gebildeten Salz, vorzugsweise Natriumchlorid und Trennen vom o-Nitrophenetol erhaltene "Mutterlauge" entspricht bei Einsatz einer ursprünglich 50%igen Lauge einer etwa 38 bis 39 %igen Lauge, vorzugsweise Natronlauge. Durch Aufkonzentrierung, beispielsweise durch Abdestillieren von Wasser im Vakuum, auf die ursprüngliche Konzentration, beispielsweise 50 % und Zusatz der dem Verbrauch entsprechenden Menge an frischer, beispielsweise 50 %iger Lauge erhält man eine in Menge und Konzentration für einen Neueinsatz geeignete Lauge. Auf diese Weise ist überraschenderweise eine abwasserfreie Herstellung von o-Nitrophenetol möglich, die praktisch nur mit dem stöchiometrischen Einsatz von Alkalimetallhydroxid und ebensolchem Salzanfall verbunden ist.

Die Umsetzung des o-Nitrochlorbenzols mit dem Ethanol erfolgt erfindungsgemäß vorzugsweise bei Temperaturen von etwa 55 bis 68 °C, insbesondere etwa 60 bis 66 °C.

Als Katalysatoren werden erfindungsgemäß Phasentransfer-Katalysatoren eingesetzt, wie sie beispielsweise in den DE-Offenlegungsschriften 2.634.419, 3.120.912 und 3.737.919 beschrieben sind. Bevorzugt sind hier die üblichen quarternären organischen Ammoniumsalze zu nennen, wie z.B. Tetrabutylammoniumbromid, Tetraethylammoniumchlorid, Tetramethylammoniumhydrogensulfat, Benzyldodecyldimethylammoniumchlorid usw. Besonders bewährt hat sich dabei Dimethylbenzylcocosalkyl-($C_{10}$-$C_{18}$)-ammoniumchlorid mit einem durchschnittlichen Molgewicht von 382,5, das bevorzugt als 50 %ige wäßrige Lösung verwendet wird. Der Katalysator kann sowohl allein, als auch im Gemisch mit anderen Phasentransfer-Katalysatoren zum Einsatz kommen, zweckmäßigerweise in einer Menge von 3 bis 15,5 Gew.-% (ca. 1,2 bis 6,2 Mol-% im Falle des besonders bevorzugten Katalysators), bezogen auf eingesetztes o-Nitrochlorbenzol, vorzugsweise 5 bis 8 Gew.-% (2,2 bis 3,3 Mol-%).

Der günstigste Reaktionsablauf in bezug auf Vollständigkeit des Umsatzes und der Vermeidung der Bildung von Nebenprodukten wird durch Zudosierung des Phasentransferkatalysators zum Reaktionsgemisch erreicht. Dies kann unabhängig von, oder zweckmäßigerweise zusammen mit der Ethanol-Zudosierung erfolgen.

Die Durchführung des erfindungsgemäßen Verfahrens verläuft zweckmäßigerweise derart, daß o-Nitrochlorbenzol zusammen mit dem wäßrigen Alkalimetallhydroxid vorgelegt und auf 62 bis 66 °C erwärmt wird. Unter wirksamem Rühren wird dann die Lösung aus Ethanol und Katalysator so zudosiert, daß die Ethanolkonzentration in der organischen Phase 5 % nicht überschreitet (Kontrolle z.B. über Gaschromatographie). Folgender Zeitplan hat sich dabei als günstig erwiesen: 50 % innerhalb von 2 Stunden, 1 Stunde nachrühren, 25 % innerhalb von 1 Stunde, 2 Stunden nachrühren, restliche 25 % in weiteren 2 Stunden. Die Zudosierzeit beträgt dann insgesamt ca. 8 Stunden. Da die Reaktion exotherm ist, muß entsprechend gekühlt werden, vor allem zu Beginn der Reaktion, wo der schnellste Reaktionsablauf stattfindet; die Temperatur sollte dabei vorzugsweise 65 bis 66 °C nicht überschreiten. Nach beendeter Dosierung rührt man nach, bis die Konzentration an nicht umgesetztem o-Nitrochlorbenzol unter 3 % gesunken ist, was im allgemeinen nach einer Nachrührzeit von 6 bis 8 Stunden der Fall ist. Es wird dann vom entstandenen Salz abfiltriert und das Filtrat in o-Nitrophenetol und "Mutterlauge" (vorzugsweise ca. 38 bis 39%ige Lauge) getrennt. Die Mutterlauge wird durch Abdestillieren von Wasser auf eine beispielsweise etwa 50 %ige Lauge aufkonzentriert und ist als solche wieder einsatzfähig.

Die nachstehenden Beispiele dienen der Erläuterung des erfindungsgemäßen Verfahrens, ohne es darauf zu beschränken.

Beispiele

1. Zu einem Gemisch aus 1264 g (8 Mol) o-Nitrochlorbenzol und 2800 g Natronlauge (50 %ig; 35 Mol), wurde bei 62 bis 66 ° C unter kräftigem Rühren eine Lösung von 420 g Ethanol (8,8 mol) und 192 g Dimethylbenzylcocosalkyl-($C_{10}$-$C_{18}$)-ammoniumchlorid (®Dodigen 226; 50 %ige wäßrige Lösung) folgendermaßen zudosiert: 306 g innerhalb von 2 Stunden, 1 Stunde nachrühren, 153 g in 1 Stunde, 2 Stunden nachrühren, restliche 153 g in 2 Stunden. Die Ethanolkonzentration lag während des gesamten Reaktionsverlaufes unterhalb von 3 Gew.-%. Um die Reaktionstemperatur von 62 bis 66 ° C aufrechtzuerhalten, wurde zu Beginn der Umsetzung gekühlt, später entsprechend geheizt. Nach beendetem Zulauf der Ethanol-/Katalysatorlösung wurde noch 6 bis 8 Stunden nachgerührt, bis der Gehalt an o-Nitrochlorbenzol kleiner als 3 % war. Danach wurde vom gebildeten Salz abfiltriert und das Filtrat im Phasenscheider in "Mutterlauge" (= 38 bis 39 %ige Natronlauge) und o-Nitrophenetol getrennt.

Nach der Phasentrennung wurden 1393 g eines katalysatorhaltigen Produkts mit einem Reingehalt von 91 % erhalten, was einer Ausbeute von 94,7 % entsprach. Das Produkt war frei von Azoxyverbindungen und o-Nitrophenol. Weiterhin erhielt man ca. 2600 g "Mutterlauge" (= 38,5 %ige NaOH), die nach Aufkonzentration durch Abdestillieren von ca. 600 g Wasser ca. 2000 g Natronlauge (50 %ig; 25 Mol) ergaben, die bei der nächsten Charge wieder eingesetzt wurden.

2. Es wurde wie im Beispiel 1 verfahren, nur daß die dort aus der Mutterlauge angefallenen 2000 g Natronlauge (50 %ig; 25 Mol) zusammen mit 800 g frischer Natronlauge (50 %ig; 10 Mol) wieder eingesetzt wurden. Der Verfahrensablauf, die Aufarbeitung und Ausbeuten entsprachen denen von Beispiel 1.

## Patentansprüche

**1.** Verfahren zur Herstellung von o-Nitrophenetol durch Umsetzung von o-Nitrochlorbenzol mit geringem Überschuß an Ethanol in 45 bis 55 gew.-%iger Alkalimetallhydroxydlösung in Gegenwart eines Phasentransfer-Katalysators bei Temperaturen von 55 bis 70 ° C, dadurch gekennzeichnet, daß die Ethanolkonzentration in der organischen Phase während des gesamten Reaktionsverlaufes 6 Gew.-%, bezogen auf die organische Phase, nicht übersteigt, daß man nach der Reaktion von gebildetem Salz abfiltriert, das Filtrat in o-Nitrophenetol und Mutterlauge trennt, und diese nach Aufkonzentrierung und Ausgleich des Verbrauchs wieder einsetzt.

**2.** Verfahren nach Anspruch 1, dadurch gekennzeichnet, daß die Ethanolkonzentration in der organischen Phase 5 Gew.-%, bezogen auf die organische Phase, nicht übersteigt.

**3.** Verfahren nach Anspruch 1 oder 2, dadurch gekennzeichnet, daß man den Phasentransfer-Katalysator und das Ethanol gleichzeitig und getrennt oder zusammen dem mit der wäßrigen Alkalimetallhydroxidlösung vorgelegten o-Nitrochlorbenzol zudosiert.

**4.** Verfahren nach mindestens einem der Ansprüche 1 bis 3, dadurch gekennzeichnet, daß man 1,05 bis 1,4 Mol Ethanol pro Mol o-Nitrochlorbenzol einsetzt.

**5.** Verfahren nach mindestens einem der Ansprüche 1 bis 4, dadurch gekennzeichnet, daß die Konzentration der Alkalimetallhydroxidlösung 47 bis 52 Gew.-% beträgt.

**6.** Verfahren nach mindestens einem der Ansprüche 1 bis 5, dadurch gekennzeichnet, daß die Umsetzung bei Temperaturen von 60 bis 66 ° C erfolgt.

**7.** Verfahren nach mindestens einem der Ansprüche 1 bis 6, dadurch gekennzeichnet, daß man als wäßrige Alkalimetallhydroxidlösung eine wäßrige Natriumhydroxidlösung oder Kaliumhydroxidlösung oder Mischungen daraus verwendet.

**8.** Verfahren nach mindestens einem der Ansprüche 1 bis 7, dadurch gekennzeichnet, daß man als Phasentransfer-Katalysator quartäre Ammoniumsalze der allgemeinen Formel verwendet

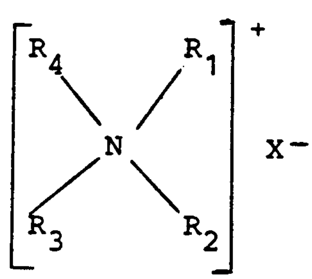

in welcher $R_1$, $R_2$, $R_3$ und $R_4$ gleiche oder verschiedene Kohlenwasserstoffreste mit einer Gesamtzahl von etwa 10 bis 50 Kohlenstoffatomen und X ein Halogenidion, Hydrogensulfation ($HSO_4$) oder Hydroxylion bedeuten.

**9.** Verfahren nach Anspruch 8, dadurch gekennzeichnet, daß man als Phasentransfer-Katalysator Dimethylbenzylcocosalkyl-($C_{10}$-$C_{18}$)-ammoniumchlorid verwendet.